# FASCICULE DE BREVET EUROPEEN

(11) **EP 0 559 509 B1**
(45) Date de publication et mention de la délivrance du brevet: **08.05.1996**
(21) Numéro de dépôt: 93400412.8
(22) Date de dépôt: 18.02.1993
(51) Int. Cl.: C07C 5/32

(54) **Procédé de déshydrogénation d'hydrocarbures aliphatiques saturés en hydrocarbures oléfiniques**
Verfahren zur Dehydrierung von aliphatischen, gesättigten Kohlenwasserstoffen zu olefinischen Kohlenwasserstoffen
Process for the dehydrogenation of aliphatic, saturated hydrocarbons to olefinic hydrocarbons

(30) Priorité: 02.03.1992 FR 9202570
(43) Date de publication de la demande: 08.09.1993
(73) Titulaire: INSTITUT FRANCAIS DU PETROLE, F-92502 Rueil-Malmaison (FR)
(72) Inventeur: Le Peltier, Fabienne, F-92500 Rueil Malmaison (FR); Robert, Sylvie, F-92500 Rueil Malmaison (FR); Boitiaux, Jean-Paul, F-78300 Poissy (FR); Leger, Gérard, F-69300 Caluire (FR); Burzynski, Jean-Pierre, F-69110 Sainte Foy Les Lyon (FR)

(56) Documents cités:
- BE-A- 568 252
- DE-B- 1 054 623
- FR-A- 1 212 491
- US-A- 3 998 900

## Description

La présente invention concerne un procédé de déshydrogénation de paraffines légères comprenant de 3 à 5 atomes de carbone par molécule utilisant un catalyseur actif pour déshydrogéner la charge caractérisé en ce qu'il est mis en oeuvre sans prémélange de la charge avec de l'hydrogène à l'entrée du réacteur. Elle concerne plus particulièrement la synthèse d'isobutène qui est utilisé notamment pour la préparation du MTBE (méthyl tertio-butyl éther) en vue de l'amélioration de l'indice d'octane dans les essences.

Il est intéressant de valoriser des hydrocarbures aliphatiques à bas point d'ébullition tels que les propanes, butanes, isobutanes, pentanes, isopentanes que l'on peut récupérer après extraction des insaturés des coupes C₃, C₄ et C₅ de vapocraquage ou de craquage catalytique, ainsi que des LPG ou des gaz de champ. Ceci justifie l'intérêt que l'on peut porter à la mise en oeuvre de procédés de conversion de ces hydrocarbures qui soient performants, sélectifs et économiques tout en contribuant également à la formation d'hydrogène.

La réaction de production d'hydrocarbures oléfiniques été décrite, notamment dans les brevets US-A- 4 381 417 et US-A- 4 381 418. Elle met en oeuvre des catalyseurs métalliques supportés à base de platine. Ces brevets décrivent un procédé régénératif dans lequel on assure une injection d'hydrogène parallèlement à l'injection d'hydrocarbures à l'entrée du réacteur. Différentes solutions visant à optimiser le recyclage de la phase riche en hydrogène sous l'angle des échanges thermiques ont été décrites. L'un des inconvénients majeurs est la nécessité de recycler tout ou partie de l'hydrogène produit ce qui complique le schéma de procédé et augmente les coûts.

Les réactions de déshydrogénation sont très rapides et réversibles. Les taux de conversion sont limités par les conditions de l'équilibre thermodynamique. Les températures élevées et les faibles pressions partielles d'hydrogène déplacent très favorablement la réaction vers la formation de composés oléfiniques. Toutefois ces conditions sévères sont très favorables à la formation de coke qui est à l'origine de la désactivation des catalyseurs de déshydrogénation. C'est pourquoi l'article "Oleflex: C2-C5 deshydrogenation Updated" de B.V. Vora, P.R. Pujado et R.F. Anderson, Energy Progress, Vol 6, N°3, 1986 pp 171 à 176 précise qu'afin de maintenir leur stabilité, les catalyseurs à base de platine sont généralement utilisés en présence d'un recyclage d'hydrogène comme le décrit notamment le brevet US-A- 3 998 900. D'autres diluants peuvent également être utilisés comme par exemple la vapeur d'eau, le méthane ou l'éthane.

D'autre part, le brevet US-A-4 962 266 décrit un procédé de préférence sans hydrogène dans la charge qui utilise un catalyseur comprenant du platine et un zincosilicalite. Ledit brevet indique la faible stabilité d'un catalyseur comprenant du platine et de l'alumine chlorée.

L'objet de l'invention concerne un procédé de déshydrogénation à partir d'une charge contenant des hydrocarbures aliphatiques saturés comprenant de 3 à 5 atomes de carbone par molécule, ledit procédé étant tel que l'on introduit la charge, préalablement chauffée à la température de réaction, sans prémélange avec de l'hydrogène à l'entrée d'au moins un réacteur dans lequel on effectue la réaction de déshydrogénation, et tel que le catalyseur comprend un support comprenant au moins un oxyde d'élément choisi parmi les groupes IIA, IIB, IIIA, IIIB, IVA et IVB de la classification périodique des éléments, au moins un métal noble de la famille du platine, au moins un métal additionnel choisi parmi les éléments du groupe VIIB ou du groupe IVA et au moins un élément alcalin ou alcalino-terreux.

Le procédé selon l'invention permet d'obtenir une conversion élevée, une sélectivité élevée en produits oléfiniques et une bonne stabilité du catalyseur. De plus, l'absence d'injection d'hydrogène à l'entrée du réacteur permet de simplifier le procédé habituellement utilisé en déshydrogénation et donc de le rendre plus économique.

Le réacteur peut comprendre un lit fixe à écoulement de gaz axial ou radial ou bien un lit mobile à écoulement radial tel que décrit dans le brevet US-A-4 277 444, ou bien encore un lit fluidisé. Un lit mobile à écoulement de gaz permettant de renouveler le catalyseur en marche est préféré. Dans ce cas, une description détaillée des réacteurs est donnée par exemple dans le brevet US-A-4 210 519 et des noyens d'assurer la circulation du catalyseur entre les différentes zones réactionnelles et la zone de régénération sont décrits par exemple dans le brevet US-A-4 981 575. Des moyens de chauffe peuvent être disposés entre les réacteurs de façon à assurer à l'entrée de chacun d'eux les conditions de température nécessaires à la réaction. La pression dans les réacteurs est maintenue aussi basse que possible, compatible avec les pertes de charge des lignes, des réacteurs et des éc'hangeurs de façon à tirer avantage de l'équilibre thermodynanique. Dans une première forme du procédé, l'effluent de la réaction de déshydrogénation (constitué des hydrocarbures aliphatiques non convertis et des produits de la réaction) est séché par exemple par passage sur un lit fixe de tamis moléculaire puis envoyé dans un séparateur dans lequel il est maintenu dans des conditions de pression et de température telles que l'on obtienne en fond une phase liquide comportant les hydrocarbures non convertis et les produits de la réaction et en tête une phase gazeuse riche en hydrogène. Les conditions de séchage sont habituellement une pression comprise entre 3.10⁵ et 3.10⁶ Pa et de préférence entre 5.10⁵ et 10⁶ Pa et une température comprise entre 10 et 100 °C, de préférence entre 30 et 60 °C. Les conditions de séparation sont habituellement une pression comprise entre 2.10⁵ et 2.10⁶ Pa, de préférence entre 4.10⁵ et 8.10⁵ Pa et une température comprise entre -10 et -120 °C et de préférence entre - 30 et -80 °C. L'hydrogène produit peut être éventuellement purifié et utilisé sur site ou bien exporté. Dans une seconde forme de procédé, l'effluent est comprimé à une pression comprise entre 2.10⁶ et 6.10⁶ Pa et à une température comprise entre -10 °C et +20 °C ce qui permet la séparation d'une phase gazeuse comportant de l'hydrogène et d'une phase liquide comprenant les hydrocarbures non convertis et les produits de la réaction. La phase gazeuse peut être purifiée ou bien utilisée telle quelle.

Dans une forme particulière du procédé il peut être avantageux d'injecter du soufre dans la charge de manière à augmenter la stabilité du catalyseur et minimiser les risques de cokage, dus à la haute température et à la présence d'oléfines, des matériaux des réacteurs, des lignes et des échangeurs inter-réacteurs. Le soufre peut par exemple être injecté sous forme de soufre organique tel que le diméthyldisulfure (DMDS) à des quantités comprises entre 1 et 200 ppm par rapport à la charge, de préférence entre 56 et 70 ppm.

La réaction de déshydrogénation s'effectue en général à une pression comprise entre 2.10⁴ et 2.10⁶ Pa et à une température comprise entre 400 et 800°C selon la nature de la charge, la température étant avantageusement comprise entre 560 et 700°C pour le propane et comprise entre 450 à 600°C pour la coupe contenant de l'isobutane, sous une pression préférée comprise entre 10⁵ et 3.10⁵ Pa. La vitesse spatiale volumique (par rapport à la charge liquide) préconisée est habituellement comprise entre 0,5 et 20 h⁻¹ et préférentiellement entre 1,5 et 6 h⁻¹.

Le catalyseur utilisé dans le procédé selon l'invention contient un support, au moins un métal noble de la famille du platine, au moins un métal additionnel, et au moins un élément alcalin ou alcalino-terreux. Les supports sont généralement choisis parmi les oxydes d'éléments choisis parmi les groupes IIA, IIB, IIIA, IIIB, IVA et IVB de la classification périodique des éléments tels que par exemple, les oxydes de magnésium, d'aluminium, de titane, de zirconium, de thorium ou de silicium pris seuls ou en mélange entre eux ou en mélange avec des oxydes d'autres éléments de la classification périodique. Le support préféré est l'alumine. La surface spécifique du support est avantageusement comprise entre 50 et 600 m² par gramme, de préférence entre 100 et 400 m² par gramme. Le métal noble est choisi parmi les éléments du groupe comprenant le platine, le palladium, le ruthénium, le rhodium, l'osmium et l'iridium. Le platine est l'élément préféré. Le métal additionnel est choisi parmi les éléments du groupe VIIB, par exemple le rhénium, ou du groupe IV A, de préférence l'étain. L'élément alcalin ou alcalino-terreux est choisi de préférence dans le groupe formé par le césium, le rubidium, le potassium, le sodium et le lithium, de préférence le lithium ou le potassium. Le catalyseur renferme en poids par rapport au support oxyde (a) de 0,01 à 2 % d'au moins un métal noble de la famille du platine (b) de 0,01 à 3 % d'au moins un métal additionnel et (c) de 0,1 à 3 % d'au moins un élément alcalin ou alcalino-terreux. La formule catalytique préférée contient de 0,1 à 1 % en poids de platine, de 0,1 à 1,5 % en poids d'étain et de 0,1 à 1,5 % en poids de potassium. Le catalyseur peut aussi contenir de 0,005 à 3,5 % en poids d'un composé halogéné tel que le chlore. Le catalyseur peut aussi contenir de 0,005 à 1 % en poids de soufre.

Le catalyseur est généralement préparé selon des méthodes classiques consistant à imprégner le support au moyen de solutions de composés des métaux que l'on désire introduire. On utilise soit une solution commune de ces métaux, soit des solutions distinctes pour le métal noble de la famille du platine, pour le métal additionnel et pour l'élément alcalin ou alcalino-terreux. Quand on utilise plusieurs solutions, on peut procéder à des séchages et/ou des calcinations intermédiaires. On termine habituellement par une calcination par exemple entre 500 et 800°C, de préférence en présence d'oxygène, par exemple en effectuant un balayage d'air.

Au moins un métal additionnel du groupe de l'étain peut être incorporé dans le support par imprégnation dudit support à l'aide d'une solution adéquate aqueuse renfermant au moins un sel du métal additionnel par exemple sous forme de chlorure, stannate, acétate ou tartrate. L'utilisation de sels organiques d'étain comme le tartrate ou l'acétate est préférée. Au moins un élément alcalin ou alcalino-terreux peut être introduit dans la masse catalytique au moyen d'une solution aqueuse contenant des sels décomposables d'au moins un élément alcalin ou alcalino-terreux sous forme de nitrate, de carbonate ou d'acétate. Par exemple de très bons résultats ont été obtenus à partir de carbonate de potassium. L'introduction d'au moins un métal noble de la famille du platine est de préférence effectuée par imprégnation du support par une solution aqueuse d'au moins un composé métallique halogéné. Le platine est généralement introduit sous forme d'acide chloroplatinique. Après l'introduction du métal noble de la famille du platine, le produit obtenu est séché puis calciné de préférence à une température comprise entre 400 et 700°C en présence d'au moins un composé organique halogéné. Parmi les composés organiques halogénés on peut citer à titre d'exemple le tétrachlorure de carbone, le chloroforme, le dichlorométane et le dichloropropane. D'une manière facultative le catalyseur peut contenir un composé du soufre. L'introduction du soufre dans le catalyseur peut être réalisée de nombreuses manières, comme par exemple, en présence d'hydrogène sulfuré dilué dans de l'hydrogène à une température comprise ente 400 et 600°C.

La charge d'hydrocarbures peut contenir des hydrocarbures saturés comprenant de 3 à 5 atomes de carbone par molécule. Ces hydrocarbures peuvent être le propane, le n-butane, le n-pentane, les isomères du butane et du pentane ou leurs mélanges.

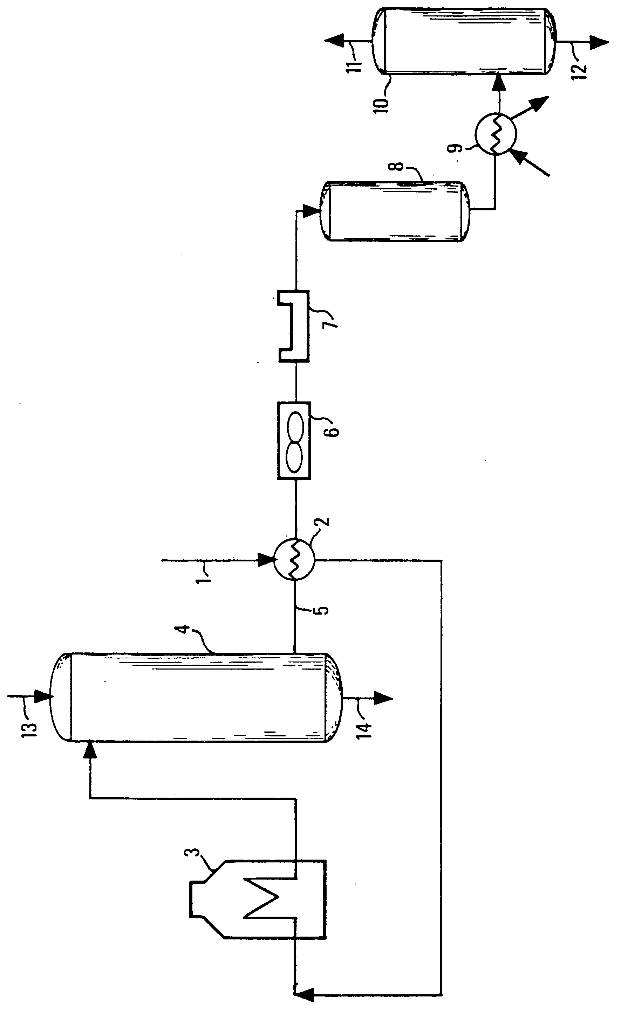

La figure jointe illustre l'invention. Elle est décrite ci-après.

Le procédé selon l'invention est généralement mis en oeuvre dans un dispos comprenant un échangeur de chaleur (2) permettant de préchauffer la charge (1) en échangeant de la chaleur avec l'effluent (5) sortant du dernier réacteur de déshydrogénation. La charge est alors dirigée vers un four (3) qui permet son préchauffage à la température de réaction avant d'être injectée dans au moins un réacteur (4) dans lequel s'effectue la réaction endothermique de déshydrogénation. Un seul réacteur a été représenté sur la figure nais le procédé selon l'invention concerne avantageusement plusieurs réacteurs de déshydrogénation. Alors, ces réacteurs sont disposés en série, l'effluent de l'un devenant la charge de l'autre. Des fours inter-réacteur permettent le réchauffage intermédiaire de l'effluent. Chaque réacteur contient un lit de catalyseur de forme annulaire en mouvement lent sous l'action de la gravité ce qui permet d'assurer le renouvellement du catalyseur. Le catalyseur frais et/ou régénéré est introduit par un conduit d'alimentation (13), tandis que le catalyseur usé est prélevé par un conduit de soutirage (14).

L'effluent de la réaction de déshydrogénation est alors dirigé par la ligne (5) vers l'échangeur de chaleur charge/effluent (2) précédemment mentionné, puis vers un aéroréfrigérant (6). L'effluent est ensuite envoyé dans un compresseur (7) et dans un sécheur, par exemple à tamis moléculaire, (8). Enfin après passage dans un échangeur (9), l'effluent est envoyé dans un séparateur (10) permettant de recueillir les hydrocarbures produits par une ligne (12) et un gaz riche en hydrogène par une ligne (11). Les hydrocarbures produits peuvent être ensuite envoyé dans une section de fractionnement (non représentée) pour être purifiée.

Les exemples qui suivent illustrent l'invention.

### EXEMPLE 1 (comparatif)

Dans cet exemple, 9,8 g d'un catalyseur dont le support est l'alumine et qui contient 0,6 % en poids de platine, 0,9 % en poids d'étain, 0,9 % en poids de potassium et 1,5 % en poids de chlore a été chargé dans un réacteur tubulaire isotherme en quartz. Ce réacteur fonctionne en flux descendant à la pression atmosphérique. Le catalyseur est calciné pendant deux heures sous un débit d'air sec de 15 l/h à 530°C avec une vitesse de montée en température de 1°C par minute. Après un balayage sous un débit d'azote, le catalyseur est ensuite réduit à 530°C sous un débit d'hydrogène de 15 l/h pendant deux heures. Ensuite on injecte pendant une heure 15 l/h d'un mélange d'hydrogène et d'hydrogène sulfuré dans un rapport molaire de 100. On termine la réduction sous 15 l/h pur à 530°C pendant une heure. Après quoi on injecte à une vitesse spatiale volumique (par rapport à la charge liquide) de 4 h⁻¹, une charge d'hydrocarbures dont la composition est précisée dans le tableau 1.

**TABLEAU 1**

| Charge | % Pds |
|---|---|
| Propylène | 0,09 |
| Propane | 1,24 |
| Isobutane | 92,57 |
| Isobutène | 0,02 |
| n-butane | 6,00 |
| n-butènes | 0,05 |
| C₅+ | 0,03 |

Durant une première période de 250 heures, on procède à une injection d'hydrogène, en co-courant de la charge d'hydrocarbures de façon à obtenir à l'entrée du réacteur un rapport molaire H₂/HC de 1. Le catalyseur se révèle être alors très stable; comme l'indique le tableau 2, le rendement en isobutène n'évolue pratiquement pas.

**TABLEAU 2**

| Durée (h) | H₂/iC₄ | T(°C) | rendement iC₄=(%pds) | conversion iC₄ (%pds) | sélectivité iC₄=(%pds) |
|---|---|---|---|---|---|
| 50 | 1 | 540 | 31,3 | 36,6 | 85,6 |
| 248 | 1 | 540 | 31,2 | 34,4 | 90,7 |

### EXEMPLE 2 (selon l'invention)

Dans cet exemple, le catalyseur est testé exactement dans les même conditions que celles décrites dans l'exemple précédent, mais, selon l'invention, la charge est introduite dans le réacteur sans prémélange avec de l'hydrogène. On observe alors qu'une conversion élevée est obtenue à une température inférieure de 30°C par rapport à celle de l'exemple 1 (510°C au lieu de 540°C). De plus, malgré les conditions sévères (H₂/iC₄=0), le catalyseur reste stable pour une nouvelle période de 150 heures.

**TABLEAU 3**

| Durée (h) | H₂/iC₄ | T(°C) | rendement iC₄=(%pds) | conversion iC₄(%pds) | sélectivité iC₄=(%pds) |
|---|---|---|---|---|---|
| 0 | 0 | 510 | 38,1 | 41,4 | 92,1 |
| 50 | 0 | 510 | 37,9 | 40,3 | 94,0 |
| 100 | 0 | 510 | 36,2 | 38,6 | 93,7 |
| 150 | 0 | 510 | 36,4 | 38,4 | 94,8 |

## Revendications

1. Procédé de déshydrogénation d'une charge comprenant des hydrocarbures aliphatiques saturés comprenant de 3 à 5 atomes de carbone par molécule, dans lequel on introduit la charge, préalablement chauffée à la température de réaction, à l'entrée d'au moins un réacteur dans lequel on effectue la réaction de déshydrogénation en présence d'un catalyseur de déshydrogénation, le procédé étant tel que la charge est introduite sans prémélange avec de l'hydrogène et tel que le catalyseur de déshydrogénation comprend un support comprenant au moins un oxyde d'élément choisi parmi les groupes IIA, IIB, IIIA, IIIB, IVA et IVB de la classification périodique des éléments, au moins un métal noble de la famille du platine, au moins un métal additionnel choisi parmi les éléments du groupe VIIB ou du groupe IVA et au moins un élément alcalin ou acalino-terreux.

2. Procédé selon la revendication 1 dans lequel le catalyseur renferme, en poids par rapport au support, de 0,01 à 2 % d'au moins un métal noble de la famille du platine, de 0,01 à 3 % d'au moins un métal additionnel et de 0,1 à 3 % d'au moins un élément alcalin ou alcalino-terreux.

3. Procédé selon l'une des revendication 1 ou 2 dans lequel ledit métal noble est le platine, ledit métal additionnel est l'étain et ledit élément alcalin est le lithium ou le potassium.

4. Procédé selon l'une des revendications 1 à 3 dans lequel le catalyseur contient de 0,1 à 1 % en poids de platine, de 0,1 à 1,5 % en poids d'étain et de 0,1 à 1,5 % en poids de potassium.

5. Procédé selon l'une des revendications 1 à 4 dans lequel le support dudit catalyseur est l'alumine.

6. Procédé selon l'une des revendications 1 à 5 dans lequel le catalyseur contient au moins un élément choisi dans le groupe formé par les composés halogénés et le soufre.

7. Procédé selon l'une des revendications 1 à 6 dans lequel la réaction de déshydrogénation s'effectue à une pression comprise entre 2.10⁴ et 2.10⁶ Pa, à une température comprise entre 400 et 800 °C et à une vitesse spatiale volumique (par rapport à la charge liquide) comprise entre 0,5 et 20 h⁻¹.

8. Procédé selon l'une des revendications 1 à 7 dans lequel l'effluent de la réaction de déshydrogénation est séché puis envoyé dans un séparateur qui permet d'obtenir une phase liquide comportant les hydrocarbures non convertis et les produits de la réaction et une phase gazeuse riche en hydrogène.

9. Procédé selon l'une des revendications 1 à 7 dans lequel l'effluent de la réaction de déshydrogénation est comprimé ce qui permet d'obtenir une phase liquide comportant les hydrocarbures non convertis et les produits de la réaction et une phase gazeuse riche en hydrogène.

10. Procédé selon l'une des revendications 1 à 9 dans lequel on injecte du soufre dans la charge.

## Claims

1. Process for the dehydrogenation of a charge incorporating saturated aliphatic hydrocarbons having 3 to 5 carbon atoms per molecule, in which the charge, previously heated to the reaction temperature, is introduced at the inlet of at least one reactor in which the dehydrogenation reaction is performed in the presence of a dehydrogenation catalyst, the process being such that the charge is introduced without premixing with hydrogen and such that the dehydrogenation catalyst comprises a support incorporating at least one oxide of an element chosen from among groups IIA, IIB, IIIA, IIIB, IVA and IVB of the periodic classification of elements, at least one noble metal from the platinum family, at least one additional metal chosen from among the elements of group VIIB or group IVA and at least one alkaline or alkaline earth element.

2. Process according to claim 1, wherein the catalyst contains, by weight based on the support, 0.01 to 2% of at least one noble metal from the platinum family, 0.01 to 3% of at least one additional metal and 0.1 to 3% of at least one alkaline or alkaline earth element.

3. Process according to either of the claims 1 and 2, wherein the noble metal is platinum, the additional metal is tin and the alkaline or alkaline earth element is lithium or potassium.

4. Process according to any one of the claims 1 to 3, wherein the catalyst contains 0.1 to 1% by weight platinum, 0.1 to 1.5% by weight tin and 0.1 to 1.5% by weight potassium.

5. Process according to any one of the claims 1 to 4, wherein the support of said catalyst is alumina.

6. Process according to any one of the claims 1 to 5, wherein the catalyst contains at least one element chosen from within the group formed by halogen-containing compounds and sulphur.

7. Process according to any one of the claims 1 to 6, wherein the dehydrogenation reaction is performed at a pressure between 2.10⁴ and 2.10⁶ Pa, at a temperature between 400 and 800°C and a volume space velocity (based on the liquid charge) between 0.5 and 20 h⁻¹.

8. Process according to any one of the claims 1 to 7, wherein the effluent of the dehydrogenation reaction is dried and then fed into a separator, which makes it possible to obtain a liquid phase incorporating the unconverted hydrocarbons and the reaction products, and a hydrogen-rich gaseous phase.

9. Process according to any one of the claims 1 to 7, wherein the dehydrogenation reaction effluent is compressed, which makes it possible to obtain a liquid phase incorporating the unconverted hydrocarbons and the reaction products, and a hydrogen-rich gaseous phase.

10. Process according to any one of the claims 1 to 9, wherein sulphur is injected into the charge.

## Patentansprüche

1. Verfahren zur Dehydrierung einer Charge, welche gesättigte, aliphatische Kohlenwasserstoffe mit 3 bis 5 Kohlenstoffatomen pro Molekül umfaßt, wobei man die auf die Reaktionstemperatur vorerhitzte Charge in den Eingang wenigstens eines Reaktors einführt, in welchem man die Dehydrierungsreaktion in Gegenwart eines Dehydrierungskatalysators durchführt, wobei das Verfahren von der Art ist, daß die Charge ohne Vorvermischung mit dem Wasserstoff eingeführt wird und daß der Dehydrierungskatalysator einen Träger umfaßt, der wenigstens ein Oxid eines Elementes, ausgewählt aus den Gruppen IIA, IIB, IIIA, IIIB, IVA und IVB des Periodensystems, wenigstens ein Edelmetall der Platinfamilie, wenigstens ein zusätzliches Metall, ausgewählt aus den Elementen der Gruppe VIIB oder IVA und wenigstens ein Alkali- oder Erdalkalimetall umfaßt.

2. Verfahren nach Anspruch 1, worin der Katalysator, in Gewicht bezogen auf den Träger, 0,01 bis 2 % wenigstens eines Edelmetalles der Platinfamilie, 0,01 bis 3 % wenigstens eines zusätzlichen Metalles und 0,1 bis 3 % wenigstens eines Alkali- oder Erdalkalimetalles umfaßt.

3. Verfahren nach einem der Ansprüche 1 oder 2, worin das Edelmetall Platin ist, das zusätzliche Metall Zinn ist und das Alkalielement Lithium oder Kalium ist.

4. Verfahren nach einem der Ansprüche 1 bis 3, worin der Katalysator 0,1 bis 1 Gew.% Platin, 0,1 bis 1,5 Gew.% Zinn und 0,1 bis 1,5 Gew.% Kalium enthält.

5. Verfahren nach einem der Ansprüche 1 bis 4, worin der Träger des Katalysators Aluminiumoxid ist.

6. Verfahren nach einem der Ansprüche 1 bis 5, worin der Katalysator wenigstens ein Element enthält, das aus der Gruppe ausgewählt ist, die durch die halogenierten Verbindungen und Schwefel gebildet ist.

7. Verfahren nach einem der Ansprüche 1 bis 6, worin die Dehydrierungsreaktion bei einem Druck, der zwischen einschließlich 2 x 10⁴ und 2 x 10⁶ Pa enthalten ist, bei einer Temperatur, die zwischen einschließlich 400 und 800°C enthalten ist und einer volumetrischen Raumgeschwindigkeit (bezogen auf die flüssige Charge), die zwischen 0,5 und 20 h⁻¹ enthalten ist, durchgeführt wird.

8. Verfahren nach einem der Ansprüche 1 bis 7, worin der Abstrom der Dehydrierungsreaktion getrocknet, dann in einen Abscheider eingeleitet wird, der es ermöglicht, eine flüssige Phase, welche die nicht umgewandelten Kohlenwasserstoffe und die Reaktionsprodukte enthält, und eine an Wasserstoff reiche gasförmige Phase zu erhalten.

9. Verfahren nach einem der Ansprüche 1 bis 7, worin der Abstrom der Dehydrierungsreaktion komprimiert wird, was es erlaubt, eine flüssige Phase, welche die nicht umgewandelten Kohlenwasserstoffe und die Reaktionsprodukte enthält, und eine an Wasserstoff reiche gasförmige Phase zu erhalten.

10. Verfahren nach einem der Ansprüche 1 bis 9, worin man der Charge Schwefel zusetzt.
